# EUROPEAN PATENT SPECIFICATION

(11) **EP 4 472 483 B1**
(45) Date of publication and mention of the grant of the patent: **24.06.2026**
(21) Application number: 23709880.1
(22) Date of filing: 06.02.2023
(51) Int. Cl.: A61B 1/005, A61B 1/008, A61B 17/29, A61M 25/01, F16L 11/15

(54) **CORRUGATED MEDICAL DEVICES**
GEWELLTE MEDIZINISCHE VORRICHTUNGEN
DISPOSITIFS MÉDICAUX ONDULÉS

(30) Priority: 08.02.2022 US 202263267700 P
(43) Date of publication of application: 11.12.2024
(73) Proprietor: Boston Scientific Scimed Inc., Maple Grove, Minnesota 55311-1566 (US)
(72) Inventor: STORBECK, Gene Thomas, Millis, Massachusetts 02054 (US); LHOTKA, Timothy, Maple Grove, Minnesota 55311 (US); GRAHAM, Shane, Fridley, Minnesota 55432 (US); MACIEJ, Matthew, Rogers, Minnesota 55374 (US); GESSLER III, Raymond David, Roberts, Wisconsin 54023 (US); PHONGSAVANH, Pat S., Blackstone, Massachusetts 01504 (US); BRECHBIEL, Scott E., Burlington, Massachusetts 01803 (US); VARADY, Marton, Shrewsbury, Massachusetts 01545 (US); POWELL, Sean, Holden, Massachusetts 01520 (US)
(74) Representative: Vossius & Partner Patentanwälte Rechtsanwälte mbB
(86) International application number: PCT/US2023/062033
(87) International publication number: WO 2023/154674

(56) References cited:
- DE-U1- 7 824 076
- GB-A- 2 056 285
- GB-A- 2 531 902
- US-A- 4 463 755
- US-A- 5 706 864
- US-A1- 2018 236 194
- US-A1- 2021 186 637

## Description

### Technical Field

This disclosure relates generally to medical devices and aspects thereof. More particularly, at least some embodiments of this disclosure relate to intruding/protruding features, e.g., corrugated features, of medical devices that may be used in various applications, e.g., endoscopic procedures, etc.

### Background

Features of medical devices used during endoscopic procedures, e.g., tubes, shafts, articulation joints, etc., generally require a specific set of properties that allow the features to function as intended, and to efficiently navigate towards the target anatomy. As a result, the aforementioned medical device features may require composite structures formed of a plurality of layers of different materials, e.g., an inner coil/tube, a surrounding layer of braiding, an outer layer, etc., as a single material generally does not encompass all of the individual properties, e.g., hoop strength, flexibility, variable flexibility, torquability, and tensile/compression properties, the medical device features may need to possess. However, manufacturing composite structures may be both costly and complex.

GB 2056285 A discloses a tracheal tube assembly that comprises an outer tube for insertion within the trachea of a patient, and an inner tube that extends within the outer tube. The inner tube is circumferentially corrugated so as to be flexible about its length and is formed with at least one groove or elongate reinforcing member traversing the corrugations.

US 4,463,755 A discloses a breathing circuit which that comprises a coaxial tube type main tube comprising an inner flexible tube constituting an inhalant circuit and a corrugated outer tube having a larger average wall thickness than said inner tube, encircling the periphery of said inner tube, and defining an exhalant circuit in conjunction with said inner tube; and an inner tube retaining member disposed at least at one end of said coaxial tube type main tube and serving to keep said inner tube and said outer tube at a prescribed distance from each other.

### Summary of the Disclosure

The present independent claim relates to a scope comprising a shaft, wherein the shaft has a corrugated body, and wherein the corrugated body includes a plurality of hoops extending around a circumference of the shaft, a plurality of indentations, wherein the plurality of indentations extend around a portion of the circumference of the shaft and define a reduced-diameter portion of the shaft relative to a diameter of the plurality of hoops, and wherein each of the plurality of hoops is spaced apart from an adjacent hoop of the plurality of hoops by at least one of the plurality of indentations, and a plurality of nodes, wherein each node extends between and connects adjacent hoops of the plurality of hoops, and wherein each of the plurality of nodes is radially offset relative to adjacent nodes along a longitudinal axis of the as claimed in claim 1. Further embodiments of the invention are defined by the dependent claims.

In at least one example, at least some of the plurality of indentations are offset from adjacent indentations by approximately 90°. Adjacent nodes of the plurality of nodes spiral from a first end of the shaft to a second end of the shaft such that adjacent nodes are offset from each other by approximately 5° to approximately 85°. In another example, adjacent nodes are offset from each other by approximately 5°. In another example, each node of the plurality of nodes is orthogonal to the adjacent hoops of the plurality of hoops that each node connects.

According to some aspects herein, each of the plurality of indentations span less than 50% of the circumference of the shaft, and wherein adjacent hoops of the plurality of hoops are separated by a pair of indentations and are connected to one another by a pair of nodes. In some aspects, each of the plurality of indentations are V-shaped radially inwards. In some aspects, each pair of indentations are offset from an adjacent pair of indentations by approximately 90°. In some aspects, the corrugated body further includes at least a first set of openings aligned along a first plane and a second set of openings aligned along a second plane, wherein the first set of openings and the second set of openings are each configured to receive a steering wire. In some aspects, the corrugated body is configured to articulate along two planes, in four different directions. In some aspects, the corrugated body is configured to articulate along two planes independently or simultaneously.

According to some aspects herein, the medical device further comprises a first end including a first connective portion and a second end including a second connective portion. In some aspects, the shaft is an articulation joint configured to be connected to an end effector. In some aspects, a pitch of each of the plurality of indentations is perpendicular relative to a longitudinal axis of the shaft. In some aspects, the corrugated body comprises a thermoplastic resin.

The present disclosure also includes a medical device comprising a corrugated shaft including a plurality of hoops extending around a circumference of the shaft, wherein each of the plurality of hoops is longitudinally offset from one another along a length of the shaft, a plurality of indentations, wherein adjacent hoops of the plurality of hoops are spaced apart from one another by a pair of indentations, wherein each of the plurality of indentations spans less than 180° around a circumference of the shaft, wherein a first indentation of a pair of indentations is separated from a second indentation of the pair of indentations by at least one node extending between and connecting adjacent hoops of the plurality of hoops, and wherein each pair of indentations is radially offset from an adjacent pair of indentations by approximately 90°. The corrugated body may further include a first set of openings aligned along a first plane and a second set of openings aligned along a second plane, wherein the first set of openings and the second set of openings are each configured to receive a steering wire. Each of the plurality of indentations may span greater than 160° but less than 180° around the circumference of the shaft. The corrugated shaft may further include a lumen and a wall surrounding the lumen, wherein a thickness of the wall is about 5 to about 30 thou.

The present disclosure also includes a medical device comprising a shaft, wherein the shaft includes a corrugated body including a plurality of hoops, a plurality of indentations, wherein each of the plurality of hoops is separated from adjacent hoops of the plurality of hoops along a length of the shaft by at least one of the plurality of indentations, and a plurality of nodes, wherein each node extends between and connects adjacent hoops of the plurality of hoops, wherein each of the indentations extends around a portion of the circumference of the shaft, wherein each of the plurality of nodes extends orthogonal to adjacent hoops of the plurality of hoops, and wherein each of the plurality of indentations is radially offset relative to adjacent indentations along the length of the shaft by approximately 90°, and adjacent nodes of the plurality of nodes spiral from a first end of the shaft to a second end of the shaft such that adjacent nodes of the plurality of nodes are radially offset from each other by approximately 5° to approximately 85° along the length of the shaft.

### Brief Description of the Drawings

The accompanying drawings, which are incorporated in and constitute a part of this specification, illustrate various exemplary embodiments and together with the description, serve to explain the principles of the disclosed embodiments.
FIGS. 1A-1B are respectively a perspective view and a cross-sectional view of a feature of a medical device, according to an embodiment.
FIG. 1C is a perspective view of a feature of a medical device, according to another embodiment.
FIGS. 2A-2B are respectively a perspective view and a cross-sectional view of a feature of a medical device, according to another embodiment.
FIGS. 3A-3B are respectively a perspective view and a cross-sectional view of a feature of a medical device, according to another embodiment.
FIGS. 4A-4B are respectively a perspective view and a cross-sectional view of a feature of a medical device, according to another embodiment.
FIGS. 5A-5C are respectively a perspective view, an elevation view, and a cross-sectional view of a feature of a medical device, according to another embodiment.
FIGS. 6A-6B are respectively a perspective view and a side view of a feature of a medical device, according to another embodiment.

### Detailed Description

Reference will now be made in detail to aspects of the disclosure, examples of which are illustrated in the accompanying drawings. Wherever possible, the same or similar reference numbers will be used through the drawings to refer to the same or like parts. The term "distal" refers to a portion farthest away from a user when introducing a device into a subject (e.g., a patient). By contrast, the term "proximal" refers to a portion closest to the user when placing the device into the subject.

Both the foregoing general description and the following detailed description are exemplary and explanatory only and are not restrictive of the features, as claimed. As used herein, the terms "comprises," "comprising," "having," "including," or other variations thereof, are intended to cover a non-exclusive inclusion such that a process, method, article, or apparatus that comprises a list of elements does not include only those elements, but may include other elements not expressly listed or inherent to such a process, method, article, or apparatus. In this disclosure, relative terms, such as, for example, "about," "substantially," "generally," and "approximately," are used to indicate a possible variation of ±10% in a stated value or characteristic.

As used herein, the term "corrugated" refers generally to a pattern of intruding and protruding features, regardless of the relative orientation of the intruding and protruding features. For example, corrugated features may or may not be parallel with one another.

Embodiments of the disclosure may solve one or more of the limitations in the art. The scope of the disclosure, however, is defined by the attached claims and not the ability to solve a specific problem. The disclosure, in certain embodiments, is drawn to medical devices including at least one corrugated feature. The medical device is not particularly limited and may be, as an example, any scope (e.g., bronchoscope, duodenoscope, endoscope, colonoscope, ureteroscope, etc.), tubing, catheter, tool, instrument, or the like, having at least one insertable, corrugated feature that extends distally from a first end towards a second end of the device. The invention as claimed in claim 1, defines a scope.

The corrugated feature may be any suitable flexible aspect of a medical device, e.g., a shaft, insertion tube, umbilicus tube, articulation joint, etc. In some examples, the corrugated feature may include at least one lumen for receiving any number of additional devices, e.g., scopes, tools, instruments, cables, fluids, or the like. The corrugated feature may be of any suitable biocompatible material that may be corrugated, and also appropriate for the intended use/function of said feature. For example, the corrugated feature may be formed of any suitable thermoplastic material, such as nylons, polyesters, fluoropolymers, polyolefins, etc.

Dimensional characteristics of the corrugated feature may vary depending on the instrument with which they are intended for use. For example, the corrugated feature may have any suitable outer diameter that may be appropriate for the feature's intended purpose. Likewise, the corrugated feature may have any suitable inner diameter, i.e., the diameter defining the lumen of the feature, that may be appropriate for the intended usage of the lumen. The outer diameter of a corrugated feature intended for insertion into larger body lumens may be larger than the outer diameter of a corrugated feature intended for insertion into narrower body lumens. Likewise, the inner diameter may depend, at least in part, on the size of the outer lumen and/or the size of the tools that must be received within the corrugated feature in order to perform a procedure. For example, the outer diameter and inner diameter of a catheter may be smaller, relative to the diameters of an umbilicus tube.

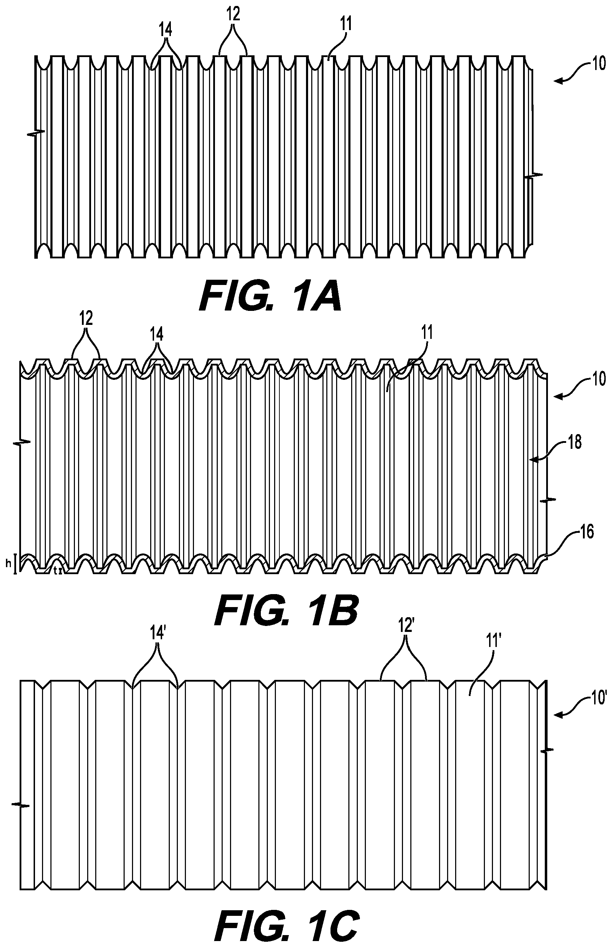

The wall thickness of the corrugated feature, i.e., the difference between the outer diameter (the diameter of the catheter) and the inner diameter (the diameter of the lumen) in a given location on the catheter, may range from about 5 thou to about 30 thou (i.e., 1/1000 of an inch. 1 inch = 2.54 cm). Wall thickness, e.g., 't' as shown in FIG. 1B, may be defined as half the difference between the inner diameter and outer diameter in a corrugated and/or non-corrugated location. The wall thickness of the corrugated feature may, along with other characteristics, define the stiffness/flexibility, torque, compression, and kink resistance of the corrugated feature. Thus, the desired level of flexibility of the corrugated feature may dictate the difference between the outer diameter and the inner diameter of the corrugated feature. It is noted that, in some examples, the wall thickness may remain consistent throughout a length of the corrugated feature, thereby maintaining a consistent level of flexibility throughout the feature. In some other examples, the wall thickness may vary throughout a length of the corrugated feature, thereby establishing a variable level of flexibility throughout the feature. For example, the wall thickness at one end may be different than the wall thickness at another end. In some aspects, the thickness of one side of the wall may be different than the thickness of another side of the wall.

The shape or contour of the corrugation, i.e., the alternating pattern of peaks and indentations, may define the various characteristics of the corrugated feature. For example, the patterning of the corrugation and the arrangement of indentations along an exterior surface of the corrugation may define the torquability of the corrugated feature. The patterning of the corrugation may include the wrapping/spiraling angle (relative to a plane perpendicular to a longitudinal axis of the feature) of a pitch that goes around the feature, down the line. Other features, such as the ratio between the width of the each of the peaks and the width of each of the indentations may also help define flexibility, as well as crushing resistance, i.e., the resistance to radial forces acting on the catheter. The corrugation height, i.e., the height of the top of the peaks from a plane extending between the bottoms of adjacent indentations, e.g., 'h' as shown in FIG. 1B, may define the hoop strength, i.e., the radial strength, of the corrugated feature, as well as stiffness, crushing resistance, and kinking resistance. In some examples, the corrugation height may be approximately 25% or less than an outer diameter of the feature, or approximately 10 to 200 thou. 1 thou = 0.0254 mm. Moreover, the shape of the indentation, e.g., circular, elliptical, V-shaped, etc., may also define directional strength and the bending mannerisms of the catheter, e.g., the bending radius, asymmetrical bending, etc. For example, if the shape of the indentation is asymmetrical, the bending radius may be the same at a lower corrugation height. If the shape of the indentation is elliptical, the bending radius may be based on the orientation of the bend relative to the radial location of the apex of the elliptical peaks.

In some examples, each of the indentations of the corrugation may be defined by two different angles, i.e., a front angle and a back angle relative to a plane bisecting across the low point of each of the indentations in a direction that is perpendicular to a longitudinal axis of the corrugated feature. The front angle may be the angle that is distal to the aforementioned plane, and the back angle may be the angle that is proximal to the aforementioned plane. It is noted that the front and back angles may also help define the degree of flexibility of the corrugated feature. Thus, flex may be increased for a given wall thickness by adjusting the front and back angles and the ratio between the width of the peaks and indentations, accordingly. Exemplary corrugation patterns are discussed further below when referring to FIGS. 1A-5C.

Corrugated features may be formed in any suitable manner. In some examples, any suitable thermoplastic, e.g., an extrusion blow molding grade thermoplastic resin, may undergo an extrusion process, thereby forming an extrudate. The extrudate may then be continuously molded into a desired corrugated shape, thereby forming a corrugated feature. The corrugated feature may be further post-processed, e.g., for the inclusion of holes, connective markings, wires, microstructures, outer protective layers, etc., to prepare the feature for its intended use. It is noted that different portions of the extrudate may be subjected to a different mold, thereby forming a plurality of different corrugations along a single extrudate. The number of different corrugations is not particularly limited. The resulting corrugated feature may thus include a number of different corrugation profiles, each of which encompass different characteristics that may be suitable for their respective functions.

In view of the above, a corrugated feature may encompass a plurality of various properties, without having to include multiple layers of various materials. Moreover, the extrusion and molding process for forming a corrugated feature may be less costly and/or simpler than manufacturing a composite structure for each individual medical device feature.

Exemplary corrugated profiles, as shown in the figures, are now further discussed. FIGS. 1A-1B illustrate a portion of an exemplary corrugated medical device feature, e.g., a shaft 10. Shaft 10 includes a plurality of hoops 11 that define peaks 12. Each of hoops 11 are adjacent to an indentation (or recess) 14, thereby establishing a repeating and alternating pattern of peaks 12 and indentations 14, as shown. Peaks 12 are planar in shape, and indentations 14 are ovular recesses or indentations, but are not limited thereto. It is noted that ovular recesses or indentations, e.g., indentations 14, may impart the ability to locate stiff sections in the radial locations that may impart different flex on different planes. Moreover, such ovular features may provide the ability to transfer forces in the axial length by locating these stiff points along the length of the corrugated aspect in such a way that it does not affect the overall flex of the corrugated aspect in terms of overall stiffness, or kink radius. Indentations 14 extend around an entire circumference of shaft 10. However, in other examples, e.g., shaft 40 and joint 50 discussed below, indentations may extend only around a partial circumference of a shaft. Shaft 10 also includes a lumen 18, defining an inner diameter, and a wall 16 surrounding lumen 18 (FIG. 1B). The dimensional characteristics, e.g., outer diameter and inner diameter, of shaft 10 is may vary, depending on the intended use of the medical device feature. For example, the inner diameter of shaft 10 may range from approximately 2 mm to approximately 30 mm. Likewise, the thickness of wall 16 may vary depending on the desired level of flexibility, and as shown, is consistent throughout a length of shaft 10, thereby maintaining consistent flexibility throughout shaft 10. In other examples, wall thickness may vary along a length of the shaft to control/vary stiffness of the shaft throughout said length. Similarly, the ratio between the width of the each of peaks 12 and the width of each of indentations 14 and the corrugation height of peaks 12 may vary depending on the desired characteristics of shaft 10. Thus, the outer diameter of shaft 10, in turn, may be defined by the sum of the inner diameter, the corrugation heights of peaks 12 (2 x h), and the thickness of wall 16 (2 x t). Shaft 10 has a symmetrical corrugation profile. Hoops 11 are at a pitch that is perpendicular to a longitudinal axis of shaft 10, and the front and back angles of indentations 14 are equivalent to one another, thereby forming a symmetrical corrugation.

FIG. 1C illustrates a shaft 10' that is similar to that of shaft 10 of FIGS. 1A-1B. However, as can be seen, shaft 10' includes hoops 11' that are of greater width, and thus, a larger ratio of the width of the each of peaks 12' to the width of each of indentations 14'. Such characteristics, in turn, may reduce the level of flexibility of shaft 10', relative to shaft 10. Furthermore, it is noted that indentations 14' define a V-groove, as opposed to the ovular shapes of indentations 14 of shaft 10. Depending on the angle of the V-groove, shaft 10' may not elongate on the outside edge of a bend, but instead foreshorten on the inside edge of the bend, thereby changing the flex characteristics of the bend geometry of shaft 10'.

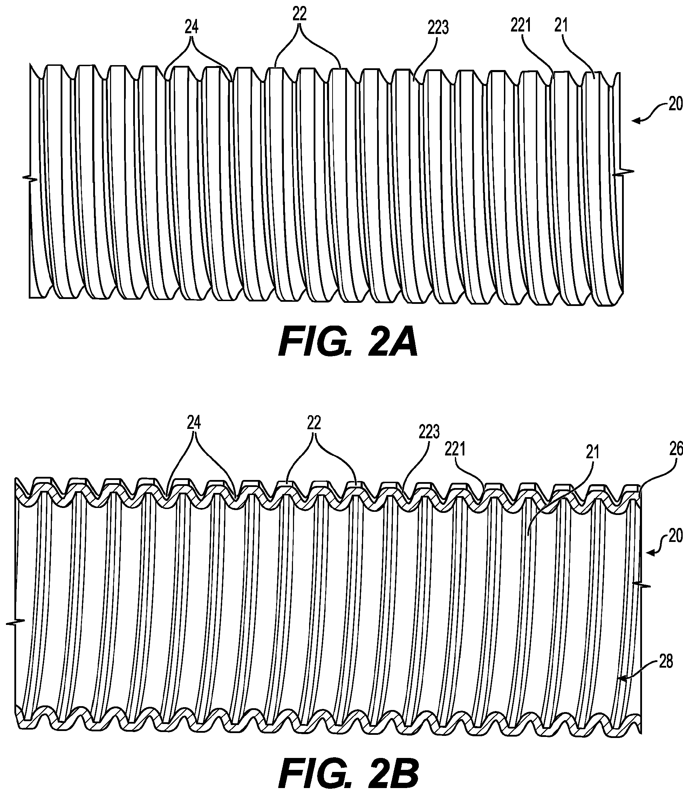

Shaft 20, as shown in FIGS. 2A-2B, is similar to shaft 10 in many respects. Like shaft 10, shaft 20 includes a plurality of hoops 21 that define peaks 22 and indentations 24. Peaks 22 are planar in shape, and indentations 24 are V-shaped indentations. Shaft 20 also includes a lumen 28 and a wall 26 surrounding lumen 28. Like shaft 10, the dimensional characteristics, e.g., outer diameter and inner diameter, the thickness of wall 26, and the ratio between the width of the each of peaks 22 and the width of each of indentations 24 and the corrugation height of peaks 22 may vary depending on the desired characteristics of shaft 20. For example, the inner diameter of shaft 20 may range from approximately 2 mm to approximately 30 mm, and the outer diameter of shaft 20, in turn, may be defined by sum the inner diameter, the corrugation height of peaks 22 (2 x h), and the thickness of wall 26 (2 x t).

Unlike shaft 10, shaft 20 may have a helical corrugation profile, i.e., a number of adjacent indentations, separated by some gap, spiraling down the longitudinal axis of shaft 20 at some angle, dictated by the aforementioned gap. Thus, hoops 21 may define a single helix spiraling down the line of shaft 20, so that hoops 21 are at a specific pitch, as indicated by line A shown in FIG. 2B, that is transverse to a plane that is perpendicular to a longitudinal axis of shaft 20, as indicated by line B. The angle between line A and line B may define a helix angle, which may vary from about 0 to about 60°, e.g., about 45°. The presence of a helix angle is indicative of each of indentations 24 defining a front angle and a back angle, as discussed above. In this example, the front angle is defined as the angle that would be formed between a first edge 221 and line B, and the back angle is defined as the angle that would be formed between a second edge 223 and line B. The front angle and the back angle are not particularly limited and may depend, at least in part, on the manufacturing method of shaft 20. In some examples, the front angle may range from about 0° to about 10°, whereas the back angle may range from about 40° to about 50°. This angled corrugation, and the number of helixes, may increase torquability of shaft 20. Furthermore, it is noted that an angled corrugation, such as that of shaft 20, may impart less flexibility, and worse bend radius, but increased compressive strength.

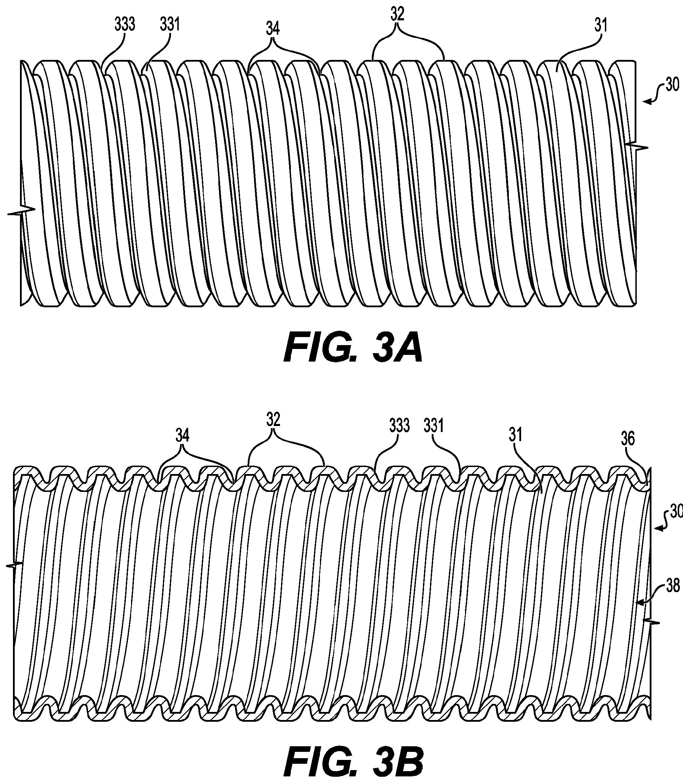

Shaft 30, as shown in FIGS. 3A-3B, is similar to shaft 20 in many respects. Like shaft 20, shaft 30 includes a plurality of hoops 31 that define peaks 32 and indentations 34, a lumen 38, and a wall 36 surrounding lumen 38.

Like shaft 20, shaft 30 also has a helical corrugation profile. However, hoops 31 define two sets of helices, i.e., a double helix, spiraling down the line of shaft 30. Hoops 31 are at a specific pitch, as indicated by line C shown in FIG. 3B, that is transverse to a plane that is perpendicular to a longitudinal axis of shaft 30, as indicated by line B. The angle between line C and line B define a helix angle, which may vary from about 0° to about 60°, e.g., about 45°. The presence of a helix angle is indicative of each of indentations 34 defining a front angle and a back angle, as discussed above. In this example, the front angle is defined as the angle that would be formed between a first edge 331 and line B, and the back angle is defined as the angle that would be formed between a second edge 333 and line B. The front angle may range from about 0° to about 10°, whereas the back angle may range from about 40° to about 50°. It is noted that because shaft 30 has a greater number of helices, relative to shaft 20, shaft 30 may have a greater degree of torquability.

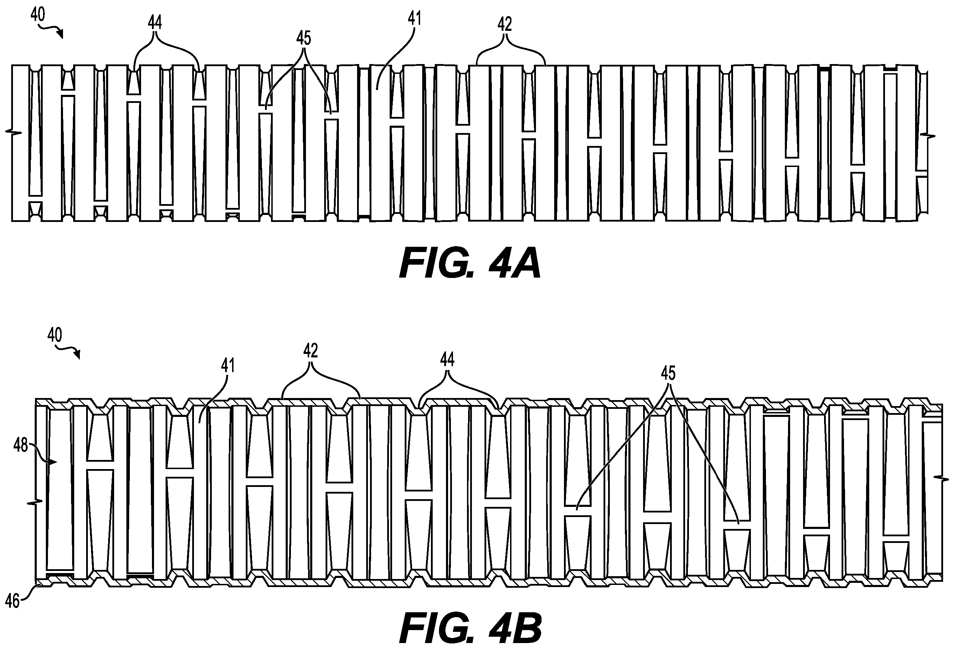

Shaft 40, as shown in FIGS. 4A-4B, is a shaft in accordance with the present claimed invention and may be similar to shaft 10. Like shaft 10, shaft 40 includes a plurality of hoops 41 that define peaks 42 and indentations 44. Peaks 42 are planar in shape. Shaft 40 also includes a lumen 48 and a wall 46 surrounding lumen 48. Like shaft 10, the dimensional characteristics, e.g., outer diameter and inner diameter, the thickness of wall 46, and the ratio between the width of the each of peaks 42 and the width of each of indentations 44 and the corrugation height of peaks 42 may vary depending on the desired characteristics of shaft 40.

Unlike indentations 14 of shaft 10, indentations 44 only extend around a partial circumference of shaft 40, thereby defining a plurality of nodes 45, i.e., the non-recessed portions interrupting indentations 44 that connect adjacent hoops 41. Nodes 45 may spiral around shaft 40 along a length of shaft 40. As shown, nodes 45 may be linear and orthogonal to hoops 41. However, it is noted that, in other examples, nodes 45 may be non-linear, and may be transverse (non-orthogonal) to hoops 41. While FIG. 4A illustrates the presence of a single node 45 bridging each indentation 44, it is noted that other examples may include a plurality of nodes 45 bridging each indentation 44 to connect adjacent hoops 41, with each nodes 45 offset from one another by a suitable degree around a circumference of shaft 40. The inclusion of nodes 45 may promote the transfer forces down the length of shaft 40 to impart a higher level of torquability without significantly affecting other characteristics.

Adjacent nodes 45 (nodes 45 in adjacent indentations 44) may be offset from each other by approximately 90°. Furthermore, adjacent nodes 45 may spiral at approximately 5° to approximately 85°, e.g., approximately 5°, relative to one another, as shown in shaft 40. Such an arrangement of nodes 45, spiraling along a length of shaft 40, may impart torsional rigidity, without imparting any significant reduction in kink. Furthermore, the above-discussed arrangement of indentations 44 and nodes 45 may improve hoop strength, tensile strength, and compressive strength, relative to corrugated shafts without nodes, due to the resulting flex of shaft 40.

It is further noted that indentations 44 may be elliptical-shaped indentations. Thus, the corrugation depth may change, while traversing around the part radially. This may be intended so that shaft 40 may separate from a mold, while imparting desired radial properties, e.g., flexibility, torquability, tensile, and compression characteristics. Moreover, such radial properties may vary along at different locations along the length of shaft 40.

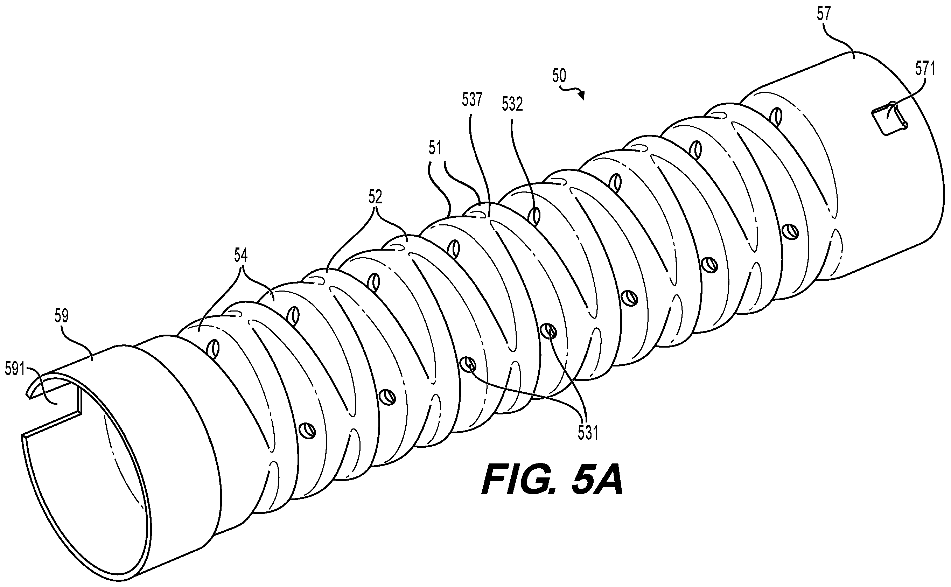

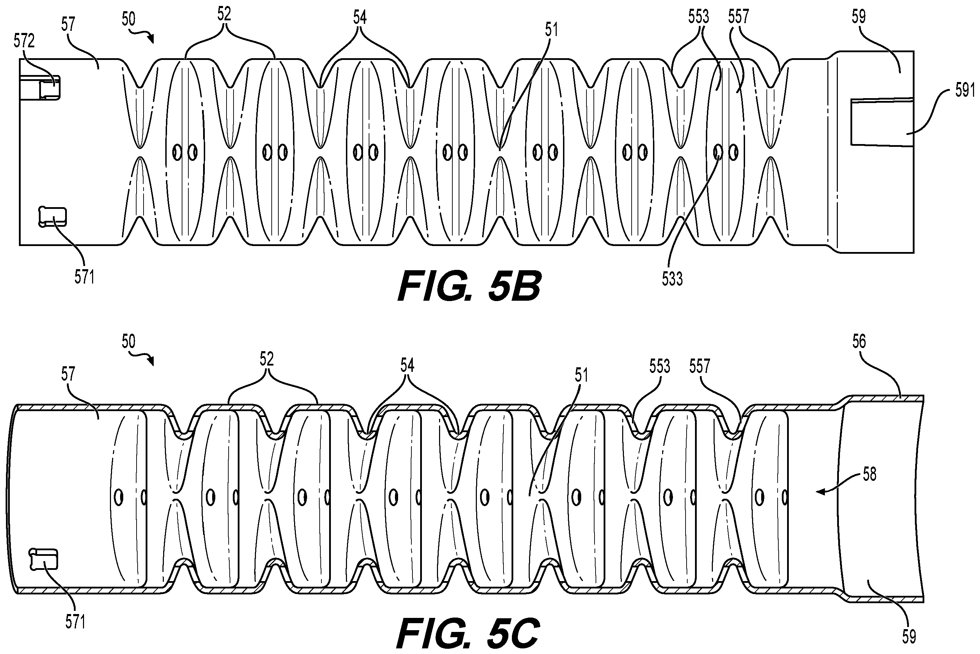

FIGS. 5A-5C illustrate an articulation joint 50, which may be similar to shaft 10 in some respects. Articulation joint 50 may be formed via any suitable means, including those requiring post-processing to impart features, e.g., holes, cutout features, etc. Like shaft 10, articulation joint 50 is tubular in shape, and includes a lumen 58 and a wall 56 surrounding lumen 58. Like shaft 10, the dimensional characteristics, e.g., outer diameter and inner diameter, the thickness of wall 56, and the ratio between the width of each of peaks 52 and the width of each of indentations 54 and the corrugation height of peaks 52 may vary depending on the desired characteristics of articulation joint 50. It is noted that the widths of the indentations 54, in combination with the depths of indentations 54, define the minimum and maximum that indentations 54 may compress or extend, thereby allowing articulation joint 50 to flex. The ratio of the width between peaks 52 and the amount of compression that each peak 52 may experience defines the radius of curvature and resulting angle of a bent articulation joint 50. **In** some examples, the bend radius of articulation joint 50 may be limited to ensure that other internal components do not kink from excessive bending.

However, unlike shaft 10, each corrugated segment of articulation joint 50 includes a pair of V-shaped indentations 54 between adjacent hoops 51. It is noted that the V-shaped indentations may vary, as the indentations may be a sharper V or a more rounded V/U shape. The shape of indentations 54 and the wall angles, e.g., the front angles and back angles as defined above, may depend on a manufacturing process of articulation joint 50. A V-shaped indentation may have more material to extend during flexing, but a U-shaped indentation may have less resistance to compress during flexing. Moreover, as shown, the front angle and back angle, as defined above, may be equal and may vary, for example, from about 0 to about 30° from perpendicular to the longitudinal axis of articulation joint 50. In some examples, the front and back angles may vary at each indentation 54, such as a distal indentation being near vertical on the distal face (i.e., a first edge 553), a proximal indentation being near vertical on the proximal face (i.e., a second edge 557), and intermediary indentations 54 having symmetric or asymmetric cross-sections.

Indentations 54 do not extend around an entire circumference (i.e., 360°) of articulation joint 50. Rather, as shown in FIG. 5A, a pair of indentations 54, between adjacent hoops 51, span around articulation joint 50 partially, e.g., less than 180° (i.e., less than 50% of the circumference), or from approximately 160° to approximately 200°, but equidistantly. As a result, the pair of indentations 54, between adjacent hoops 51, may be separated by non-recessed aspects 537, which also bridge peaks 52 of the adjacent hoops 51. It is noted that non-recessed aspects may be of varying thickness, depending on the degree by which indentations 54 span around a circumference of articulation joint 50. Such thickness may vary the stiffness/flexibility of articulation joint 50. For example, thicker portions may result in a stiffer articulation joint. It is also noted that each pair of indentations 54, between adjacent hoops 51, may be in a 90° phase relative to one another, as shown in FIG. 5A. As a result of indentations 54 being in alternating phase, articulation joint 50 may compress and extend along two planes, independently from one another or simultaneously with one another, thereby allowing steering/articulation of articulation joint 50 in two directions.

The benefits of a corrugated articulation joint, e.g., articulation joint 50, include but are not limited to, being manufactured in one piece, and potentially the same piece as an insertion tube, not requiring expensive injection molds, not relying on thin breakable section of molded plastic that flex during articulation, and not being subjected to over-articulation as a result of individual segments shifting and overlapping.

Articulation joint 50 further includes a plurality of openings - a first set of openings 531, a second set of openings 532, a third set of openings 533 (shown in FIG. 5B), and a fourth set of openings (not shown) along each of a first edge 553 of indentations 54 and a second edge 557 of indentations 54. Each set of the four sets of openings are configured to receive a respective steering wire. Thus, openings 531 may be along the same plane, and likewise, openings 532, 533, and the fourth set of openings, not shown, may be along their respective planes as well. Each of the aforementioned planes of openings 531, 532, 533, and the fourth set of openings may be approximately 90° offset from one another, thereby allowing for articulation/steering along the two planes as discussed above, and four directions (e.g., up, down, left, and right). In an alternate embodiment, openings 531 and 533 with their respective indentations 54 may be omitted, thereby allowing articulation/steering in only a single plane as discussed above (e.g., up and down, or left and right).

Articulation joint 50 also includes a first end 57, which includes a first connective portion 571 and a second connective portion 572, and a second end 59, including a third connective portion 591. Connective portions 571, 572, and 591 are not particularly limited, and may be any suitable feature configured to engage an adjacent medical device aspect or feature, e.g., a shaft, end-effector, etc. For example, more or fewer connective portions may be included at each end, and the connectors may take different forms, sizes, and/or shapes depending on the connection mechanism required to affix articulation join 50 to a cooperating feature or medical device aspect. Other exemplary joints may include additional or less connective portions, or may be without such connection portions altogether.

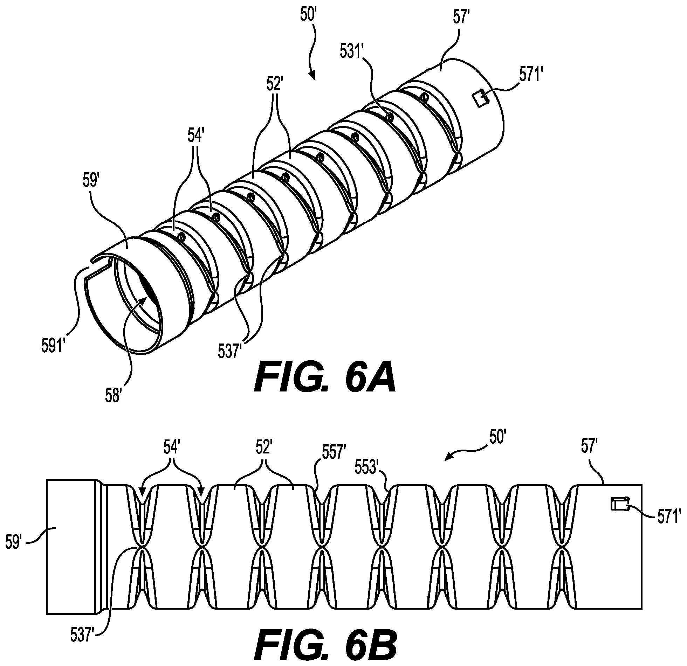

In an alternate embodiment, alternate indentations may be approximately 0° from one another resulting in an articulation joint that may only bend in a single plane. Such an alternate embodiment, i.e., articulation joint 50', is illustrated in FIGS. 6A and 6B.

Articulation joint 50' is similar to articulation joint 50 in many respects, and like reference numerals refer to like parts. As noted above, articulation joint 50' include a plurality of indentations 54', where each pair of adjacent indentations 54' are approximately 0° relative to one another. Thus, articulation joint 50' may compress and extend along only along a single plane. In view of this, articulation joint 50' may only include two sets of openings, a set of openings 531' and another set of openings (not shown), along each of first edge 553' and second edge 557' of indentations 54'. Openings 531' and the other set of openings are configured to receive a respective steering wire, thereby allowing for articulation/steering along the single plane, as discussed above, and two directions (e.g., up and down, or left and right, depending on the orientation of articulation joint 50').

It will be apparent to those skilled in the art that various modifications and variations can be made to the disclosed device without departing from the scope of the disclosure. For example, although embodiments of the disclosure focus mainly on articulation in two planes, as discussed above, embodiments may be modified to articulate in a single plane, or in more than two planes. Accordingly, embodiments of the disclosure are intended to articulate in at least one plane. Other embodiments of the disclosure will be apparent to those skilled in the art from consideration of the specification and practice of the invention disclosed herein. It is intended that the specification and examples be considered as exemplary only, with a true scope of the invention being indicated by the following claims.

## Claims

1. A scope, comprising:
a shaft (10, 20, 30, 40), wherein the shaft (10, 20, 30, 40) has a corrugated body, and wherein the corrugated body includes:
a plurality of hoops (11, 21, 31, 41) extending around a circumference of the shaft (10, 20, 30, 40);
a plurality of indentations (14, 24, 34, 44, 54), wherein the plurality of indentations (14, 24, 34, 44, 54) extend around a portion of the circumference of the shaft (10, 20, 30, 40) and define a reduced-diameter portion of the shaft (10, 20, 30, 40) relative to a diameter of the plurality of hoops (11, 21, 31, 41), and wherein each of the plurality of hoops (11, 21, 31, 41) is spaced apart from an adjacent hoop (41, 51) of the plurality of hoops (11, 21, 31, 41) by at least one of the plurality of indentations (14, 24, 34, 44, 54); and
a plurality of nodes (45), wherein each node (45) extends between and connects adjacent hoops (41, 51) of the plurality of hoops (11, 21, 31, 41), and
wherein each of the plurality of nodes (45) is radially offset relative to adjacent nodes (45) along a longitudinal axis of the shaft (10, 20, 30, 40).

2. The scope of claim 1, wherein at least some of the plurality of indentations (14, 24, 34, 44, 54) are offset from adjacent indentations (14, 24, 34, 44, 54) by approximately 90°.

3. The scope of claims 1 or 2, wherein adjacent nodes (45) of the plurality of nodes spiral from a first end (57) of the shaft (10, 20, 30, 40) to a second end (59) of the shaft (10, 20, 30, 40) such that adjacent nodes (45) are offset from each other by approximately 5° to approximately 85°.

4. The scope of claim 3, wherein adjacent nodes (45) are offset from each other by approximately 5°.

5. The scope of claim 3, wherein each node (45) of the plurality of nodes (45) is orthogonal to the adjacent hoops (41, 51) of the plurality of hoops (11, 21, 31, 41) that each node (45) connects.

6. The scope of any of the preceding claims, wherein each of the plurality of indentations (14, 24, 34, 44, 54) span less than 50% of the circumference of the shaft (10, 20, 30, 40), and wherein adjacent hoops (41, 51) of the plurality of hoops (11, 21, 31, 41) are separated by a pair of indentations (14, 24, 34, 44, 54) and are connected to one another by a pair of nodes (45).

7. The scope of claim 6, wherein each of the plurality of indentations (14, 24, 34, 44, 54) are V-shaped radially inwards.

8. The scope of claim 6, wherein each pair of indentations (14, 24, 34, 44, 54) are offset from an adjacent pair of indentations (14, 24, 34, 44, 54) by approximately 90°.

9. The scope of claim 8, wherein the corrugated body further includes at least a first set of openings aligned along a first plane and a second set of openings aligned along a second plane, wherein the first set of openings and the second set of openings are each configured to receive a steering wire.

10. The scope of claim 9, wherein the corrugated body is configured to articulate along two planes, in four different directions.

11. The scope of claim 9, wherein the corrugated body is configured to articulate along two planes independently or simultaneously.

12. The scope of any of the preceding claims, further comprising a first end (57) including a first connective portion (571) and a second end (59) including a second connective portion (591).

13. The scope of any of the preceding claims, wherein a pitch of each of the plurality of indentations (14, 24, 34, 44, 54) is perpendicular relative to a longitudinal axis of the shaft (10, 20, 30, 40).

14. The scope of any of the preceding claims, wherein the corrugated body comprises a thermoplastic resin.

## Patentansprüche

1. Skop, das aufweist:
einen Schaft (10, 20, 30, 40), wobei der Schaft (10, 20, 30, 40) einen geriffelten Körper hat und wobei der geriffelten Körper aufweist:
mehrere Ringe (11, 21, 31, 41), die sich um einen Umfang des Schafts (10, 20, 30, 40) erstrecken;
mehrere Vertiefungen (14, 24, 34, 44, 54), wobei sich die mehreren Vertiefungen (14, 24, 34, 44, 54) um einen Abschnitt des Umfangs des Schafts (10, 20, 30, 40) erstrecken und einen mit reduziertem Durchmesser ausgebildeten Abschnitt des Schafts (10, 20, 30, 40) relativ zu einem Durchmesser der mehreren Ringe (11, 21, 31, 41) definieren und wobei jeder der mehreren Ringe (11, 21, 31, 41) von einem benachbarten Ring (41, 51) der mehreren Ringe (11, 21, 31, 41) durch mindestens eine der mehreren Vertiefungen (14, 24, 34, 44, 54) beabstandet ist; und
mehrere Verbindungen (45), wobei sich jede Verbindungen (45) zwischen benachbarten Ringen (41, 51) der mehreren Ringe (11, 21, 31, 41) erstreckt und sie verbindet und
wobei jede der mehreren Verbindungen (45) relativ zu benachbarten Verbindungen (45) entlang einer Längsachse des Schafts (10, 20, 30, 40) radial versetzt ist.

2. Skop nach Anspruch 1, wobei mindestens einige der mehreren Vertiefungen (14, 24, 34, 44, 54) von benachbarten Vertiefungen (14, 24, 34, 44, 54) um etwa 90° versetzt sind.

3. Skop nach Anspruch 1 oder 2, wobei sich benachbarte Verbindungen (45) der mehreren Verbindungen von einem ersten Ende (57) des Schafts (10, 20, 30, 40) zu einem zweiten Ende (59) des Schafts (10, 20, 30, 40) so spiralförmig winden, dass benachbarte Verbindungen (45) um etwa 5° bis etwa 85° voneinander versetzt sind.

4. Skop nach Anspruch 3, wobei benachbarte Knoten (45) um etwa 5° voneinander versetzt sind.

5. Skop nach Anspruch 3, wobei jede Verbindung (45) der mehreren Verbindungen (45) orthogonal zu den benachbarten Ringen (41, 51) der mehreren Ringe (11, 21, 31, 41) ist, die jede Verbindungen (45) verbindet.

6. Skop nach einem der vorstehenden Ansprüche, wobei jede der mehreren Vertiefungen (14, 24, 34, 44, 54) weniger als 50 % des Umfangs des Schafts (10, 20, 30, 40) überspannt und wobei benachbarte Ringe (41, 51) der mehreren Ringe (11, 21, 31, 41) durch ein Paar Vertiefungen (14, 24, 34, 44, 54) getrennt und durch ein Paar Verbindungen (45) miteinander verbunden sind.

7. Skop nach Anspruch 6, wobei jede der mehreren Vertiefungen (14, 24, 34, 44, 54) radial nach innen V-förmig ist.

8. Skop nach Anspruch 6, wobei jedes Paar Vertiefungen (14, 24, 34, 44, 54) von einem benachbarten Paar Vertiefungen (14, 24, 34, 44, 54) um etwa 90° versetzt ist.

9. Skop nach Anspruch 8, wobei der geriffelten Körper ferner mindestens einen ersten Satz von Öffnungen, die entlang einer ersten Ebene ausgerichtet sind, und einen zweiten Satz von Öffnungen aufweist, die entlang einer zweiten Ebene ausgerichtet sind, wobei der erste Satz von Öffnungen und der zweite Satz von Öffnungen jeweils so konfiguriert sind, dass sie einen Lenkdraht aufnehmen.

10. Skop nach Anspruch 9, wobei der geriffelten Körper so konfiguriert ist, dass er sich entlang zweier Ebenen in vier unterschiedlichen Richtungen gelenkig bewegt.

11. Skop nach Anspruch 9, wobei der geriffelten Körper so konfiguriert ist, dass er sich entlang zweier Ebenen unabhängig oder gleichzeitig gelenkig bewegt.

12. Skop nach einem der vorstehenden Ansprüche, das ferner ein erstes Ende (57) mit einem ersten Verbindungsabschnitt (571) und ein zweites Ende (59) mit einem zweiten Verbindungsabschnitt (591) aufweist.

13. Skop nach einem der vorstehenden Ansprüche, wobei eine Steigung jeder der mehreren Vertiefungen (14, 24, 34, 44, 54) relativ zu einer Längsachse des Schafts (10, 20, 30, 40) senkrecht ist.

14. Skop nach einem der vorstehenden Ansprüche, wobei der geriffelten Körper ein thermoplastisches Harz aufweist.

## Revendications

1. Scope comprenant :
une tige (10, 20, 30, 40), dans lequel la tige (10, 20, 30, 40) a un corps crénelé, et dans lequel le corps crénelé comprend :
une pluralité d'anneaux (11, 21, 31, 41) s'étendant autour d'une circonférence de la tige (10, 20, 30, 40) ;
une pluralité d'indentations (14, 24, 34, 44, 54), dans lequel la pluralité d'indentations (14, 24, 34, 44, 54) s'étendent autour d'une partie de la circonférence de la tige (10, 20, 30, 40) et définissent une partie de diamètre réduit de la tige (10, 20, 30, 40) par rapport à un diamètre de la pluralité d'anneaux (11, 21, 31, 41) et dans lequel chacun de la pluralité d'anneaux (11, 21, 31, 41) est espacé d'un anneau (41, 51) adjacent de la pluralité d'anneaux (11, 21, 31, 41) selon au moins l'une de la pluralité d'indentations (14, 24, 34, 44, 54) ; et
une pluralité de nœuds (45), dans lequel chaque nœud (45) s'étend entre et raccorde les anneaux (41, 51) adjacents de la pluralité d'anneaux (11, 21, 31, 41), et
dans lequel chacun de la pluralité de nœuds (45) est radialement décalé par rapport aux nœuds (45) adjacents le long d'un axe longitudinal de la tige (10, 20, 30, 40).

2. Scope selon la revendication 1, dans lequel au moins certaines de la pluralité d'indentations (14, 24, 34, 44, 54) sont décalées des indentations (14, 24, 34, 44, 54) adjacentes d'approximativement 90°.

3. Scope selon les revendications 1 ou 2, dans lequel les nœuds (45) adjacents de la pluralité de nœuds décrivent une spirale d'une première extrémité (57) de la tige (10, 20, 30, 40) à une deuxième extrémité (59) de la tige (10, 20, 30, 40) de sorte que les nœuds (45) adjacents sont décalés les uns des autres d'approximativement 5° à approximativement 85°.

4. Scope selon la revendication 3, dans lequel les nœuds (45) adjacents sont décalés les uns des autres d'approximativement 5°.

5. Scope selon la revendication 3, dans lequel chaque nœud (45) de la pluralité de nœuds (45) est orthogonal aux anneaux (41, 51) adjacents de la pluralité d'anneaux (11, 21, 31, 41) que chaque nœud (45) raccorde.

6. Scope selon l'une quelconque des revendications précédentes, dans lequel chacune de la pluralité d'indentations (14, 24, 34, 44, 54) couvre moins de 50% de la circonférence de la tige (10, 20, 30, 40) et dans lequel les anneaux (41, 51) adjacents de la pluralité d'anneaux (11, 21, 31, 41) sont séparés par une paire d'indentations (14, 24, 34, 44, 54) et sont raccordés entre eux par une paire de nœuds (45).

7. Scope selon la revendication 6, dans lequel chacune de la pluralité d'indentations (14, 24, 34, 44, 54) sont en forme de V radialement vers l'intérieur.

8. Scope selon la revendication 6, dans lequel chaque paire d'indentations (14, 24, 34, 44, 54) est décalée d'une paire adjacente d'indentations (14, 24, 34, 44, 54) d'approximativement 90°.

9. Scope selon la revendication 8, dans lequel le corps crénelé comprend en outre au moins un premier ensemble d'ouvertures aligné le long d'un premier plan et un deuxième ensemble d'ouvertures aligné le long d'un deuxième plan, dans lequel le premier ensemble d'ouvertures et le deuxième ensemble d'ouvertures sont chacun configurés pour recevoir un fil de direction.

10. Scope selon la revendication 9, dans lequel le corps crénelé est configuré pour s'articuler le long de deux plans, dans quatre directions différentes.

11. Scope selon la revendication 9, dans lequel le corps crénelé est configuré pour s'articuler le long de deux plans de manière indépendante ou simultanée.

12. Scope selon l'une quelconque des revendications précédentes, comprenant en outre une première extrémité (57) comprenant une première partie de connexion (571) et une deuxième extrémité (59) comprenant une deuxième partie de connexion (591).

13. Scope selon l'une quelconque des revendications précédentes, dans lequel un pas de chacune de la pluralité d'indentations (14, 24, 34, 44, 54) est perpendiculaire par rapport à un axe longitudinal de la tige (10, 20, 30, 40).

14. Scope selon l'une quelconque des revendications précédentes, dans lequel le corps crénelé comprend une résine thermoplastique.
